# EUROPEAN PATENT APPLICATION

(11) **EP 4 151 544 A1**
(43) Date of publication of application: **22.03.2023**
(21) Application number: 21197229.4
(22) Date of filing: 16.09.2021
(51) Int. Cl.: B65B 55/18, B65B 35/16, B65B 9/13, B65B 41/16, B65B 51/30, B65B 43/30, B65B 57/16, B65B 55/02, A61B 90/70, A61L 2/24, A61L 2/025, A61L 2/18, B08B 1/00, B08B 1/04, B08B 11/02, A61C 19/00, B25J 9/00, B25J 5/02, B25J 11/00, B25J 9/16, G05B 19/414

(54) **APPARATUS FOR STERILIZING AND PACKAGING DENTAL AND/OR SURGICAL INSTRUMENTS**

(71) Applicant: Castagno, Giorgio Giacomo, 13011 Borgosesia VC (IT)
(72) Inventor: Castagno, Giorgio Giacomo, 13011 Borgosesia VC (IT)
(74) Representative: Barbaro, Gaetano

(57) **Abstract**

This disclosure illustrates an equipment for sterilizing and packaging dental and / or surgical instruments that allows the task of sterilization and packaging of dental and / or surgical tools to be performed in an automated manner. The equipment includes in particular a robotic arm with at least one gripper, configured to grasp, move and release dental and / or surgical instruments; at least one device for washing, cleaning and disinfecting, at least one device for sterilizing dental and / or surgical instruments; a container for recognizing dental and / or surgical instruments; a packaging machine; a motorized trolley that may be moved linearly forward / backward, on which a base of the robotic arm is fixed; at least one microprocessor control unit, configured to perform at least one of the following operations: commanding forward / backward movements of the motorized trolley, commanding movements of the robotic arm, recognizing the identification code on dental and / or surgical instruments, operating the plurality of mobile suction devices.

## Description

### TECHNICAL FIELD

The present disclosure refers in general to sterilization equipment and more particularly to an equipment for sterilizing and packaging dental and / or surgical instruments.

### BACKGROUND

Typically, dental and / or surgical manual instruments ("tools") are decontaminated / disinfected by immersing them in a tank containing water and solvents, then applying ultrasounds to stir the water in order to wash the "tools" by removing residues of biological material, or by placing them in a thermodisinfector. The instruments are then taken from the tank and dried, or from the thermodisinfector, then placed in a sealed package and treated in an autoclave for final sterilization.

A medical office with several doctors may receive even more than a hundred patients a day, so many tools are used every day, which must be taken from sealed and perfectly sterile bags. Once used, the tools are collected indiscriminately and there is a specialized assistant who has the sole task of collecting the used dental tools, sterilizing them, separating them and making the bags sealed with sterile tools available to doctors. This person is therefore employed full time in repetitive and risky operations to his/her health, since he/she is exposed to potentially pathogen-contaminated tools from several patients. The recovery procedure of the used dental tools is also exposed to human error, which could cause, for example, the imperfect disinfection of the tools with the consequent risk of transmission of pathogens from one patient to another. Furthermore, it is sometimes advisable to package two or more instruments together used to perform a single operation on the patient, in order to reduce waste of sealed bags and to optimize the use of the autoclave, in which the sealed bags not overlapping each other must be inserted for the sterilization of the contained tools. Any errors in packing multiple tools together would force to re-sterilize a tool as soon as the sealed package is broken, even if it has not actually been used on the patient.

It would therefore be desirable to have a sterilization and packaging apparatus suitable for dental and / or surgical instruments, which allows to obviate at least some of the aforementioned problems.

### SUMMARY

An equipment has been created, as defined in the attached claims, to sterilize and package dental and / or surgical instruments that allows the task of sterilizing and packaging of dental and / or surgical tools to be performed in an automated manner. The equipment includes in particular a robotic arm with at least one gripper, configured to grasp, move and release dental and / or surgical instruments; at least one device for sterilizing dental and / or surgical instruments; a container for recognizing dental and / or surgical instruments; a packaging machine; a motorized trolley that may be moved linearly forward / backward, on which a base of said robotic arm is fixed; at least one microprocessor control unit, configured to perform at least one of the following operations: commanding forward / backward movements of the motorized trolley, commanding movements of the robotic arm, visually recognizing dental and / or surgical instruments, operating the plurality of mobile suction devices.

According to an embodiment, the dental and / or surgical instruments bear an identification code and the microprocessor control unit is configured to recognize the dental and / or surgical instruments by reading the relative identification code.

According to an embodiment, the device for sterilizing dental and / or surgical instruments has at least one cleaning / disinfection and drying tank.

According to an embodiment, the equipment comprises: a first microprocessor control unit, configured to control the forward / backward movements of the motorized trolley and to control the movements of the robotic arm; a second microprocessor control unit, configured to activate / deactivate the device for sterilization; a third microprocessor control unit, configured to recognize dental and / or surgical instruments; a fourth microprocessor control unit, configured to operate the plurality of mobile suction devices; each microprocessor control unit of the microprocessor control unit being interfaced with the other microprocessor control units to signal the beginning and an end of a task to be carried out / carried out.

Optionally, the apparatus may be equipped with a self-propelled vehicle suitable for transporting a container for dental and / or surgical instruments, the vehicle being configured to carry out a forward / backward path from a first pick-up location for used dental and / or surgical instruments, to a second place where the robotic arm is installed.

Advantageously, the self-propelled vehicle will have a fifth microprocessor control unit configured to operate the vehicle itself and to signal a beginning and interfaced with the other microprocessor control units to signal the end of a task to be carried out / carried out.

Other embodiments are defined in the attached claims.

### BRIEF DESCRIPTION OF THE DRAWINGS

Figure 1 schematically illustrates an exemplary embodiment of an apparatus according to the present disclosure to sterilize and package dental and / or surgical instruments in a fully automated manner.
Figure 2 is a front view of the equipment of Figure 1.
Figure 3 is a top view of the equipment in figure 1.
Figure 4 is a detail view of four mobile suction devices, configured to suck an edge of a plastic package fed by a continuous roll and to move it to open or close the plastic package.

### DETAILED DESCRIPTION

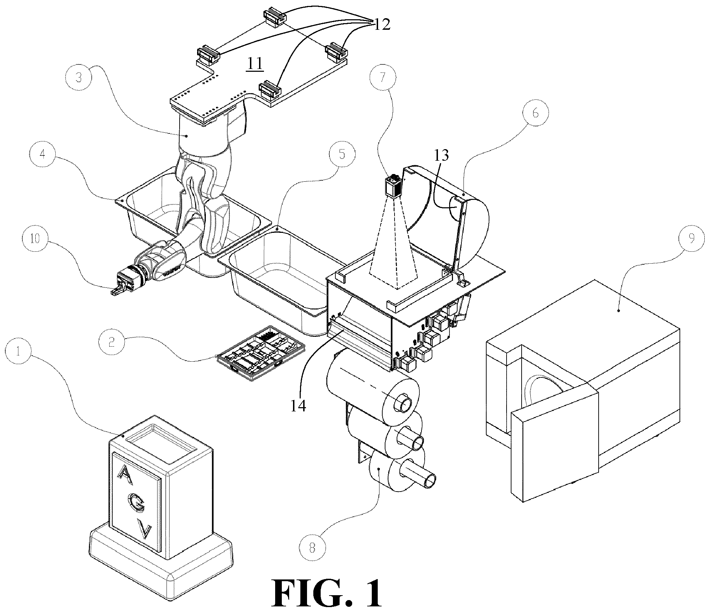

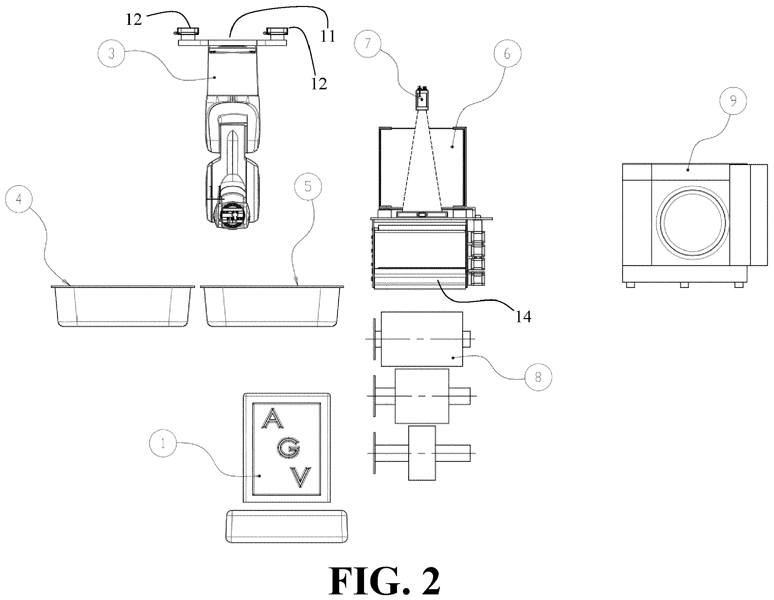

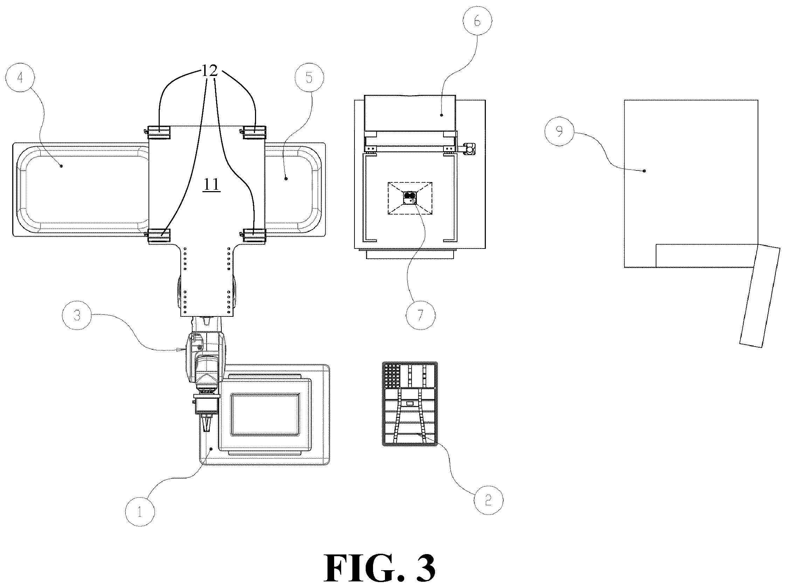

An exemplary embodiment of an apparatus of this disclosure for sterilizing and packaging dental and / or surgical instruments within sealed and sterile plastic packages is shown in the perspective view of Figure 1 and in the front and top views of the Figures 2 and 3. It includes various devices that interact with each other in a coordinated manner so as to perform the daily work of a person in charge of sterilization and packaging of dental and / or surgical tools in a dental office or clinic in a fully automated manner, or more generally in any environment where sterilization is required.

According to one aspect, the robotic arm 3 equipped with a gripper 10 has a base fixed to a motorized carriage 11, which may translate it linearly back and forth along a linear guide (not shown). To this end, the carriage may be provided with one or more slides 12, for example four illustrated slides 12 to slide on two tracks (not shown). The robotic arm 3 is articulated and is configured to grasp, move and release dental and / or surgical instruments. The equipment of this disclosure has at least one device for sterilizing dental and / or surgical instruments.

According to an aspect shown in the figure, the device for sterilizing dental and / or surgical instruments has at least one washing / disinfection and drying tank 4, 5.

According to another aspect, not illustrated in the figures, the washing / disinfection device is configured for rotary / ultrasonic instruments, such as for example handpieces for dentists, which notoriously cannot be immersed in disinfectant liquids. In this case, the washing / disinfection device comprises means for mechanically cleaning the handpieces, such as opposed motorized rollers equipped with bristles, preferably mixed hardness bristles, so that any residues present on the handpieces are mechanically removed by rubbing the bristles of the motorized rollers against the handpieces to be cleaned.

Preferably, but not necessarily, during the cleaning operation with the motorized rollers, the handpiece to be cleaned will be solidly anchored to the robotic arm, which will move and rotate it according to predetermined movements so as to expose all its parts to the action of the roller bristles. Preferably, but not necessarily, the rollers will be partially immersed in a solution based on disinfectant liquid so that the bristles are wet with disinfectant liquid before being rubbed against the handpieces.

According to an aspect not shown in the figures, the washing / disinfection device is configured in such a way as to have a quick coupling socket to hold the handpiece, which has been mechanically cleaned from the bristles of the motorized rollers, while through an internal duct of the washing / disinfection device a pressurized fluid is injected into the cleaned handpiece. This pressurized fluid will contain specific detergents for dental handpieces, of the type currently used when performing this task manually, in order to sanitize and lubricate the internal parts of the cleaned handpiece.

In the exemplary embodiment shown in the figures, the device for sterilizing comprises a tank or tanks 4, 5, which may be of the ultrasonic type operating at adjustable power on the basis of a feedback probe and with a drying system, and next to the tank o tanks 4, 5 there is a visual recognition device for dental and / or surgical instruments, which has:
- a tray 2 on which to store dental and / or surgical instruments,
- a dome cover 6 with an apical opening 13, the dome cover 6 being functionally hinged to pass from a closed position, in which it covers the tray 2, to an open position (shown in the figures) in which it releases the tray 2,
- at least one image acquisition device 7 configured to visually recognize the dental and / or surgical instruments.

According to one aspect, the visual recognition device of dental and / or surgical instruments performs "image processing" methods to recognize the contours of dental and / or surgical instruments in a captured image and to isolate from the captured image only the pixels that belong to the same dental and / or surgical instrument, as well as "pattern recognition" algorithms to identify the type of dental and / or surgical instrument that has been seen.

According to a preferred embodiment, each one of the dental and / or surgical instruments carries a respective identification code and the image acquisition device 7 is configured to read the identification code imprinted on the dental and / or surgical instruments, so as to facilitate recognition of each instrument.

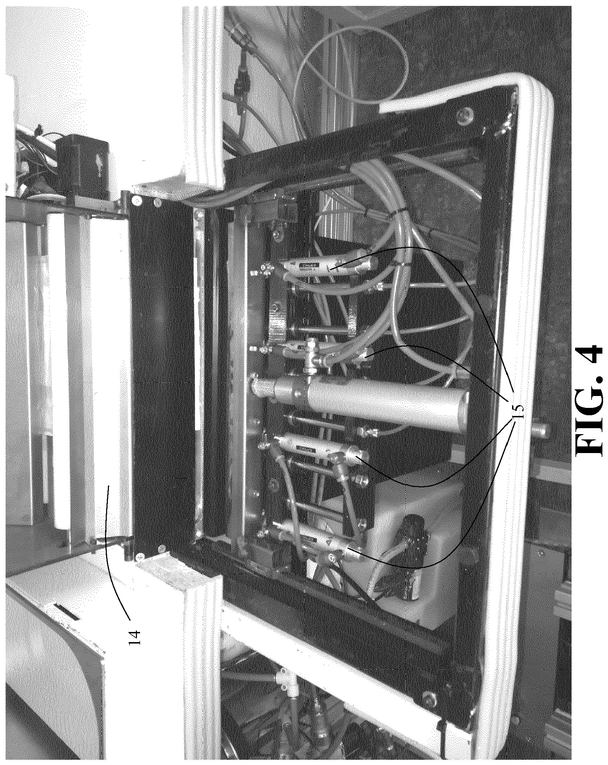

In the vicinity of the recognition device, conveniently at the bottom, vertically with respect to the position of tray 2, there is a packaging machine 14, 15, comprising:
- a plurality of mobile suction devices 15, shown in the photograph of figure 4, configured to suck an edge of a plastic package fed by a continuous roller 8 and to move it so as to open or close the plastic package,
- a sealing device 14, configured to seal the edge of the plastic package and to detach it from the respective roller 8.

The activities of the various parts of the equipment are coordinated by at least one microprocessor control unit (not shown), configured to perform at least one of the following operations:
- commanding forward / backward movements of the motorized trolley 11,
- commanding movements of the robotic arm 3,
- recognizing the identification code on the dental and / or surgical instruments,
- operating the mobile suction devices 15 in an appropriate manner according to need.

According to a particularly advantageous optional aspect, the apparatus of the present disclosure may be equipped with a self-propelled vehicle 1 suitable for transporting a container for dental and / or surgical instruments, configured to carry out a forward / backward path from a first place for picking up used dental and / or surgical instruments, to a second place where the robotic arm 3 is installed. When there is the need to sterilize surgical instruments, the self-propelled vehicle 1, indicated in the figures with the abbreviation AGV which means "Automated Guided Vehicle", is called by an operator. When the vehicle reaches its destination, the trays 2 with the contaminated material are placed inside the AGV. The self-propelled vehicle 1 AGV leaves the room where it was called and transports its contents to the sterilization laboratory.

When reaching the sterilization laboratory, the AGV enters and heads towards the unloading area and the robotic arm 3 picks up the content, placing it in a support area.

When the robotic arm 3 handles dirty tools, it uses a non-sterile gripper 10.

The robotic arm 3 introduces the used tools into the device to sterilize. For example, with reference to the exemplary embodiment shown in the figures, it inserts them in at least one washing / disinfection and drying tank 4, 5. In the embodiment shown in the figures, there are two sterilization and drying tanks 4, 5, which may be, for example, ultrasonic tanks or an ultrasonic double tank with adjustable power operating on the basis of a feedback probe and with a drying system. Once the tanks 4, 5 are closed with the tools to be sterilized inside, the robotic arm 3 changes the coating of the gripper 10, which was used to handle non-sterile objects, wearing a disinfected cover for handling objects who have already passed the first stages of sterilization.

According to one aspect, the tank or tanks (as shown in the figure) are ultrasonic tanks with adjustable power with infrared light for drying. Tanks of this type that may be used in the apparatus of this disclosure may be of the ultrasonic type with adjustable power and with operation based on a feedback probe and with a drying system. According to one aspect, the apparatus of this disclosure may use a thermodisinfector, for example of the type currently used to sterilize dental instruments.

Once the washing / disinfection inside these tanks 4, 5 is completed, the detergent liquid is discharged in the usual manner and a washing with demineralized water is carried out.

Whether the dental and / or surgical instruments are suitable to be immersed in water and have been washed in the tanks, or whether these instruments are handpieces that have been mechanically cleaned by two motorized rollers with bristles, the clean instruments are dried, for example infrared drying.

Referring only as an example to the embodiment shown in the figures, when the tanks 4, 5 open, the robotic arm 3 picks up the disinfected and dried tools using the pliers 10, covered with the disinfected coating, and passes them to the recognition device. The tools will be placed on a tray 2 so that the image acquisition device 7 may recognize them. For example, if the tools all have a respective identification code, they will be placed on the tray so that the respective identification code may be read by the image acquisition device 7.

According to one aspect, for example, the identification code associated with dental and / or surgical instruments is of the data matrix type. According to one aspect, this identification code is indelibly stamped on an external surface of each instrument itself when the instrument is manufactured. Alternatively, if an instrument does not have an identification code stamped by the manufacturer, then a ring may be made with an identification code stamped on it and the ring may be inserted around the instrument itself.

In order to facilitate the reading of the identification code by the image acquisition device 7, according to one aspect, the recognition device may be equipped with a dome cover 6 with an apical opening 13, the dome cover 6 being functionally hinged to switch from a closed position, in which it covers the tray 2, to an open position in which it releases the tray 2. In this way, an illuminated environment is created in an ideal way for reading, for example, data matrix on tools placed on the tray 2, with the image acquisition device 7 facing above the dome cover 6 to read through the apical opening 13 the identification code imprinted on the dental and / or surgical instruments placed on the tray 2.

After reading and identifying the tools, the dome cover 6 opens and if a dental and / or surgical instrument is to be wrapped, the robotic arm 3 passes it to the packaging machine 14, 15, otherwise this phase is skipped. For the packaging operation, the roll 8 will be activated to slide the continuous plastic film, which will be blocked by the mobile suction devices 15 according to the desired length.

According to one aspect, the mobile suction devices 15 may be of the type with pistons and suction cups as shown in Figure 4.

A photocell (not shown in the figure) communicates when to block the sliding of the plastic film. Once the plastic film has been blocked at the desired length, the sealing device 14 is activated, which may typically be a hot blade, and seals what will be the lower part of the package. At this point the continuous roller 8 starts to turn again to make the film unwind, up to the desired length, blocking it again. The plastic film is cut, the suction cups open the bag and the tools are placed inside the package, which is sealed at the bottom and still open at the top. The sealing device 14 (the hot blade) is activated again to seal the upper part of the package, the suction cups are released and the pistons that held the package still are released, and a sealed package is obtained with the tools inside.

Figure 4 shows four pistons 15 of the packaging machine and three continuous rollers 8 which unwind plastic films of different widths to make packages of different sizes. According to one aspect, a piston 15 will always be activated regardless of the width of the plastic film, and a second piston 15 among the other three will be chosen on the basis of the width of the plastic film to be unwound from the respective continuous roll 8, so as to cooperate with the first piston 15 to open the package. If it is desired to provide for the possibility of having packages with more than three different widths, conveniently there will be N different continuous rolls 8 and N + 1 pistons, one of which is always activated and one, chosen among the others N, activated depending on the size of the chosen plastic film.

Finally, the bags and objects are placed on another tray that will be inserted inside the autoclave 9, whose door is automated. From this moment, the robotic arm 3 changes the disinfected covering with the sterile ones. At the end of the autoclave cycle, while the robotic arm 3 picks up the trays from the autoclave 9, the self-propelled AGV vehicle 1 is called in the sterilization room. The robotic arm 3 extracts the trays from the autoclave 9 and passes to the AGV vehicle 1, which will leave the room to bring the sterilized content back to where the initial call started.

According to one aspect, the apparatus of this disclosure comprises a first microprocessor control unit, configured to control the forward / backward movements of the motorized carriage and to control the movements of the robotic arm 3, a second microprocessor control unit, configured for activate / deactivate said device for sterilization, a third microprocessor control unit, configured to recognize the identification code on dental and / or surgical instruments, and a fourth microprocessor control unit, configured to operate the mobile suction devices. In addition, each microprocessor control unit is interfaced with the other microprocessor control units to signal the beginning and the end of a task to be carried out / carried out. Thanks to this configuration, a single control unit is no longer required to supervise all operations, which may currently be done with relatively high cost units. The different microprocessor control units must control the operations of the specific device only, independently of the other devices that make up the equipment. To this end, the Arduino^{™} type microprocessor units may even be used, which have a very low cost and are easily programmable.

In the embodiments which provide for the presence of the self-propelled vehicle 1 AGV, conveniently there will also be a fifth microprocessor control unit configured to operate the self-propelled vehicle 1 and interfaced with the other microprocessor control units to signal the completion of a task to be carried out / carried out.

According to one aspect, the self-propelled device 1 AGV will have: a lower part, which deals with the movement of the vehicle, an upper part that has a compartment area for waste collection (optional), and another area for collecting trays on where the surgical instruments have been placed. The latter area will, for example, be divided into two parts: a loading area for non-sterilized tools, and an area for loading sterilized tools that have already completed the sterilization cycle.

According to one aspect, the self-propelled vehicle 1 will have a display for calling, checking the system status, and viewing the patient's medical record.

According to one aspect, the self-propelled vehicle 1 will be equipped with a voice command device (eg Alexa^{™} or Google Home^{™}) to call the self-propelled vehicle 1 AGV via voice message.

Returning to the example of the dental office, the first goal of the project is the elimination of the human presence in a biologically dangerous environment. The equipment of this disclosure is completely capable of replacing the figure of the instrument nurse as part of the sterilization process because it may perform the same functions continuously in the same precise way, while a human operator may make mistakes. The equipment thus guarantees total safety of the service, with the possibility of certifying that the tools have actually been sterilized according to standards. It also achieves the elimination of the risks of contracting infections both for patients and for the personnel assigned to the transfer of dental and / or surgical instruments and decontamination, thus obtaining a higher level in the quality of the proposed service and the standards of the entire structure.

Thanks to the recognition device integrated inside the equipment, it is possible to monitor the status of the tools. This translates into a better awareness of wear, and therefore of their durability, of each individual tool, so as to be able to use it at its maximum working cycle. In addition, the equipment of this disclosure provides information on the actual number of present tools, always achieving real control of the warehouse, avoiding possible waste (e.g. a human operator could unknowingly throw away a tool, even of a high value). The equipment recognizes the objects that will have to undergo the sterilization process thanks to the identification code. Furthermore, it is possible to optimize the cycle times of the autoclave 9. The robotic arm 3 will also be able to arrange the different packages on the trays intended for sterilization, optimizing the use of the internal spaces in the autoclave and positioning the packages correctly, keeping between them a sufficient space to allow the normal heat flow for an even more homogeneous sterilization. The consequence deriving from this continuous workflow is energy saving.

## Claims

1. An apparatus for sterilizing and packaging dental and / or surgical instruments, comprising:
a robotic arm (3) with at least one grasping forceps (10), configured to grasp, move and release dental and / or surgical instruments;
at least one device for sterilizing dental and / or surgical instruments;
at least one visual recognition device for dental and / or surgical instruments, comprising:
- at least one tray (2) on which to store dental and / or surgical instruments,
- at least one image acquisition device (7) configured to read the identification code on the dental and / or surgical instruments placed on the tray (2);
a packaging machine (14, 15), comprising:
- a plurality of mobile suction devices (15), configured to suck an edge of a plastic package fed by a continuous roll (8) and to move it so as to open or close the plastic package,
- a sealing device (14), configured to seal the edge of the plastic package and to detach it from the respective roll (8);
a motorized trolley (11), linearly movable forward / backward along a linear guide, on which a base of said robotic arm is fixed;
at least one microprocessor control unit, configured to perform at least one of the following operations: command forward / backward movements of the motorized trolley (11), command movements of the robotic arm (3), visually recognize the dental and / or surgical instruments, operating said plurality of mobile suction devices (15).

2. The apparatus according to claim 1, wherein:
said dental and / or surgical instruments each carry an identification code;
said image acquisition device (7) is configured to read the identification code imprinted on the dental and / or surgical instruments placed on the tray (2);
said microprocessor control unit is configured to recognize the identification code on the dental and / or surgical instruments.

3. The apparatus according to claim 1 or 2, comprising:
a first microprocessor control unit, configured to control the forward / backward movements of the motorized trolley (11) and to control the movements of the robotic arm (3);
a second microprocessor control unit, configured to activate / deactivate said device for sterilizing;
a third microprocessor control unit, configured to recognize the identification code on the dental and / or surgical instruments;
a fourth microprocessor control unit, configured to actuate said plurality of mobile suction devices (15);
each microprocessor control unit of said microprocessor control units being interfaced with the other microprocessor control units to signal a beginning and a completion of a task to be carried out / just carried out.

4. The apparatus according to one of claims 1 to 3, comprising a self-propelled vehicle (1) suitable for transporting a container for dental and / or surgical instruments, said vehicle (1) being configured to travel forward / backwards along a path from a first pickup place of used dental and / or surgical instruments, to a second place where the robotic arm is installed (3).

5. The apparatus according to claim 4, comprising a fifth microprocessor control unit configured to actuate said self-propelled vehicle (1) and interfaced with the other microprocessor control units to signal a start / completion of a task to be carried out / just carried out.

6. The apparatus according to one of the preceding claims, wherein:
said recognition device comprises a dome cover (6) with an apical opening (13), the dome cover (6) being functionally hinged to pass from a closed position, in which it covers the tray (2), to an open position in which it releases the tray (2);
said image acquisition device (7) facing above the dome cover (6) to visually recognize the dental and / or surgical instruments placed on the tray (2).

7. The apparatus according to the preceding claims, wherein said image acquisition device (7) is configured to read through the apical opening (13) an identification code placed on the dental and / or surgical instruments placed on the tray (2).

8. The apparatus according to one of the preceding claims, wherein said device for sterilizing comprises at least a washing-disinfecting and drying tank (4, 5) and at least one device between a thermo-disinfector with said washing-disinfecting and drying tank (4, 5) and a ultrasound device which irradiates ultrasounds in said washing-disinfecting and drying tank (4, 5) when the tank is filled with water.

9. The apparatus according to one of the preceding claims, wherein said packaging machine (14, 15) comprises three dispensers of plastic film rolls (8) of different sizes and includes at least four suction devices (15) independent of each other, a first suction device of said suction devices (15) being configured to be always activated each time a package must be opened / closed, only one of the other suction devices being activated at a time together with said first suction device according to an opening size of the package to be opened / closed.

10. The apparatus according to one of the preceding claims, comprising at least an autoclave (9) configured to accommodate internally sealed packages of dental instruments and / or surgical equipment and to perform a final sterilization.

11. The apparatus according to one of the preceding claims, wherein said image acquisition device (7) is configured to read a data matrix type code.

12. The apparatus according to one of the preceding claims, comprising at least one of the following characteristics from a) to e):
a) a plurality of said robotic arms (3) with at least one grasping forceps (10), each configured to grip, move and release dental and / or surgical instruments, as many motorized trolleys (11), each movable linearly forward / backward along a linear guide, on which a base of a respective robotic arm is fixed, each robotic arm being equipped with a first microprocessor control unit, configured to command forward / backward movements of the respective motorized trolley (11) and to command movements of the respective robotic arm (3);
b) a plurality of said devices for sterilizing dental and / or surgical instruments, each having at least one sterilization and drying tank and having a respective second microprocessor control unit configured to activate / deactivate the respective device for sterilizing;
c) a plurality of said devices for recognizing dental and / or surgical instruments bearing an identification code, each device for recognizing having a respective third microprocessor control unit configured to recognize the identification code on the dental and / or surgical instruments;
d) a plurality of said packaging machines (14, 15), each equipped with a respective fourth microprocessor control unit, configured to operate the respective mobile suction devices (15);
e) a plurality of self-propelled vehicles (1), each suitable for transporting a container for dental and / or surgical instruments, each vehicle (1) being configured to carry out a forward / backward path from a first place of collection of used dental and / or surgical instruments, to a second place where said at least one robotic arm (3) is installed, each self-propelled vehicle (1) being equipped with a respective fifth microprocessor control unit configured to operate the respective self-propelled vehicle (1);
wherein all said microprocessor control units are interfaced with each other to signal a beginning / completion of a task to be carried out / just carried out.
